(19) Europäisches Patentamt
European Patent Office
Office européen des brevets

(11) Veröffentlichungsnummer: **0 064 256**
**B1**

(12) # EUROPÄISCHE PATENTSCHRIFT

(45) Veröffentlichungstag der Patentschrift: **12.03.86**

(51) Int. Cl.⁴: **C 07 D 501/36**, A 61 K 31/545

(21) Anmeldenummer: **82103536.7**

(22) Anmeldetag: **27.04.82**

(54) **Cephemderivate und Verfahren zu ihrer Herstellung.**

(30) Priorität: **02.05.81 DE 3117438**

(43) Veröffentlichungstag der Anmeldung: **10.11.82 Patentblatt 82/45**

(45) Bekanntmachung des Hinweises auf die Patenterteilung: **12.03.86 Patentblatt 86/11**

(84) Benannte Vertragsstaaten: **AT BE CH DE FR GB IT LI NL**

(56) Entgegenhaltungen:
**FR - A - 2 385 722**

**UNLISTED DRUGS, Band 33, Nr. 11, November 1981, page 174, Sub. e "cefodizime"**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Mencke, Burkhard, Dr., Im Güldenstück 13, D-6270 Idstein/Taunus (DE)**
Erfinder: **Ehlers, Eberhard, Dr., Hundshager Weg 2, D-6238 Hofheim am Taunus (DE)**
Erfinder: **Blumbach, Jürgen, Dr., Merziger Weg 1a, D-6000 Frankfurt am Main 71 (DE)**
Erfinder: **Dürckheimer, Walter, Dr., Im Lerchenfeld 45, D-6234 Hattersheim am Main (DE)**
Erfinder: **Seeger, Karl, Dr., Schwalbenweg 9, D-6238 Hofheim am Taunus (DE)**

**EP 0 064 256 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Gegenstand der Erfindung sind Cephemderivate der allgemeinen Formel I

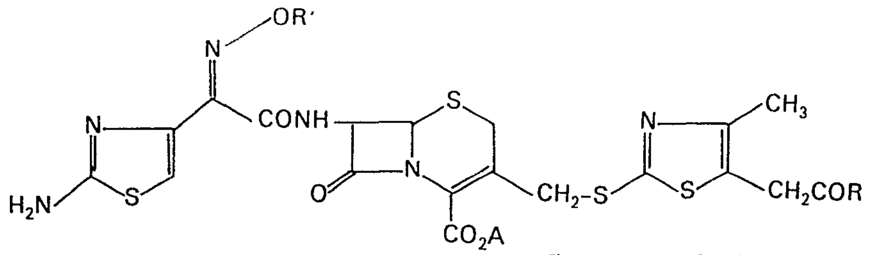

I

in der R die Bedeutung besitzt
von Alkoxy mit 1–4 C-Atomen, das substituiert sein kann durch Alkoxy mit 1–4 C-Atomen oder Phenyloxy,
von Alkenoxy mit 2–3 C-Atomen, das substituiert sein kann durch Phenyl,
von Alkinoxy mit 2–3 C-Atomen,
von Phenyloxy, das substituiert sein kann durch Halogen,
Alkyl mit 1–4 C-Atomen oder Alkoxy mit 1–4 C-Atomen,
von Phenylalkoxy mit 1–2 C-Atomen im Alkylteil, wobei der Phenylteil substituiert sein kann durch Halogen, Alkyl mit 1–4 C-Atomen oder Alkoxy mit 1–4 C-Atomen,
von Amino,
von Alkyl- oder Dialkylamino mit 1–4 C-Atomen pro Alkylteil,
von Phenylamino, wobei der Phenylteil substituiert sein kann durch Halogen, Alkyl mit 1–4 C-Atomen oder Alkoxy mit 1–4 C-Atomen,
R′ steht für Alkyl mit 1–4 C-Atomen und
A für Wasserstoff, ein physiologisch unbedenkliches Kation oder eine physiologisch unbedenkliche Estergruppe und in der die R′O-Gruppe in syn-Position steht.

Die vorliegende Erfindung betrifft somit Verbindungen der allgemeinen Formel I, in der die Substituenten beispielsweise die folgende Bedeutung besitzen können.

R kann stehen für
Alkoxy mit 1 bis 4 C-Atomen, das substituiert sein kann durch Alkoxy mit 1 bis 4 C-Atomen, oder Phenyloxy, für
Alkenoxy mit 2 bis 3 C-Atomen, das substituiert sein kann durch Phenyl, für
Alkinoxy mit 2 bis 3 C-Atomen, für
Phenyloxy, das substituiert sein kann durch Halogen, insbesondere Chlor und Brom, Alkyl mit 1 bis 4 C-Atomen, Alkoxy mit 1 bis 4 C-Atomen, für
Phenylalkoxy mit 1 bis 2 C-Atomen im Alkylteil, wobei der Phenylteil substituiert sein kann durch Halogen, vorzugsweise Chlor und Brom, Alkyl mit 1 bis 4 C-Atomen, Alkoxy mit 1 bis 4 C-Atomen, für Amino, für
Alkylamino mit 1 bis 4 C-Atomen im Alkylteil, für
Dialkylamino mit 1 bis 4 C-Atomen pro Alkylteil, für
Phenylamino, wobei der Phenylteil substituiert sein kann durch Halogen, insbesondere Chlor und Brom, Alkyl mit 1 bis 4 C-Atomen oder Alkoxy mit 1 bis 4 C-Atomen.

Im folgenden seien einige Gruppen aufgeführt, die erfindungsgemäss für die Substituenten R, R′ und A von besonderem Interesse sind.

Steht R für gegebenenfalls substituiertes Alkoxy mit 1 bis 4 C-Atomen, so seien insbesondere genannt Methoxy, Äthoxy, Propoxy, iso-Propoxy, Butoxy, iso-Butoxy, tert.-Butoxy, Methoxyäthoxy, Äthoxyäthoxy, Phenoxyäthoxy, wobei besonders bevorzugt sind, Methoxy, Äthoxy, iso-Propoxy, iso-Butoxy, tert.-Butoxy, Methoxyäthoxy und Phenoxyäthoxy.

Steht R für gegebenenfalls substituiertes Alkenoxy, so seien als besonders bevorzugt genannt Allyloxy und Cinnamyloxy.

Bei R in der Bedeutung von Alkinoxy ist Propargyloxy besonders bevorzugt.

Steht R für gegebenenfalls substituiertes Phenyloxy, so seien insbesondere genannt Phenoxy, 4-Tolyloxy, 2-Chlor- und 4-Chlorphenoxy, 3,4-Dichlorphenoxy, 4-Methoxyphenoxy, 3,4-Dimethoxyphenoxy, wobei besonders bevorzugt sind Phenoxy, 4-Chlorphenoxy, 3,4-Dichlorphenoxy, 4-Methoxyphenoxy und 3,4-Dichlormethoxyphenoxy.

R in der Bedeutung von Phenylalkoxy kann beispielsweise stehen für Benzyloxy, 4-Methylbenzyloxy, 4-Chlorbenzyloxy, 4-Methoxybenzyloxy, 3,4-Dimethoxybenzyloxy und Phenäthoxy, bevorzugt jedoch für Benzyloxy, 4-Chlorbenzyloxy, 3,4-Dichlorbenzyloxy, 4-Methoxybenzyloxy und 3,4-Dimethoxybenzyloxy.

R in der Bedeutung von Alkylamino kann z.B. stehen für Methylamino, Äthylamino, Propylamino oder Butylamino, bevorzugt jedoch für Methylamino und Äthylamino.

Steht R für Dialkylamino, so seien als Beispiele genannt Dimethylamino, Diäthylamino, Dipropylamino, Diisopropylamino, bevorzugt jedoch Dimethylamino und Diäthylamino.

R in der Bedeutung von gegebenenfalls substituiertem Phenylamino kann z.B. stehen für Anilino, 4-Tolylamino, 4-Chlorphenylamino, 4-Methoxyphenylamino, 2-Methoxyphenylamino, bevorzugt jedoch für Anilino, 4-Chlorphenylamino und 4-Methoxyphenylamino.

Steht R′ für Alkyl mit 1 bis 4 C-Atomen, so sind bevorzugt Methyl, Äthyl, n-Propyl, iso-Propyl, n-Butyl, iso-Butyl, besonders bevorzugt jedoch Methyl.

Steht A für einen Rest, der den Charakter einer Schutzgruppe trägt, so seien beispielsweise als bevorzugt erwähnt tert.-Butyl und Trimethylsilyl, sowie auch Benzyl, Benzhydryl, Trichloräthyl-4-methoxybenzyl oder 4-Nitrobenzyl.

Steht A für eine physiologisch verträgliche Estergruppe, so kommt z.B. 1-Acyloxyalkyl mit 1 bis 6, vorzugsweise 1 bis 4 C-Atomen sowohl im Acyl- als auch im Alkylteil in Betracht, wie z.B. Acetoxymethyl, 1-Acetoxyäthyl, 1-Acetoxypropyl, 1-Acetoxyisopropyl, 1-Acetoxyhexyl, Propionyloxymethyl, 1-Propionyloxyäthyl, 1-Propionyloxypropyl, 1-Propionyloxyhexyl, 1-Pivaloyloxymethyl, 1-Pivaloyloxyäthyl, insbesondere jedoch Acetoxymethyl, 1-Acetoxyäthyl, 1-Propionyloxyäthyl und Pivaloyloxymethyl.

Als physiologisch unbedenkliche Kationen seien beispielsweise genannt Alkaliionen, insbesondere das Natrium- und Kaliumion, Erdalkaliionen, insbesondere das Calcium- und Magnesiumion, sowie ein Ammoniumion, vorzugsweise jedoch ein Natriumion, sowie ein alkyliertes Ammoniumion, wobei ein Alkylrest 1 bis 4 C-Atome haben kann, wie insbesondere Triäthylammonium, Diäthylammonium, Dimethylammonium oder Morpholinium.

Gegenstand der Erfindung ist weiterhin ein Verfahren zur Herstellung von Cephemverbindungen der allgemeinen Formel I, das dadurch gekennzeichnet ist, dass man Lactam der allgemeinen Formel II

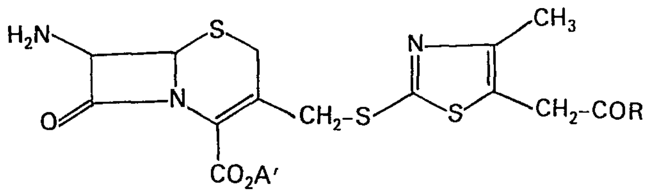

II

in der R die oben angegebene Bedeutung hat und A′ für ein Kation, eine Schutzgruppe oder einen physiologisch unbedenklichen Ester steht, mit einer Carbonsäure der allgemeinen Formel III

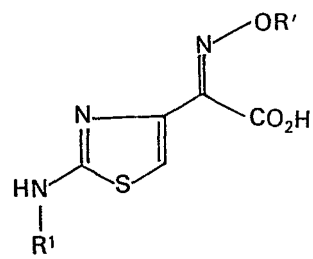

III

oder einem reaktionsfähigen Derivat derselben, in der $R^1$ für Wasserstoff oder eine aus der Peptidchemie bekannte Aminoschutzgruppe steht und R′ die obige Bedeutung besitzt, zu einer Verbindung der allgemeinen Formel IV

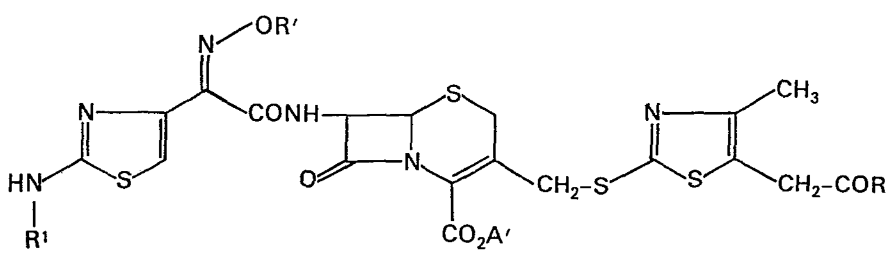

IV

in der $R^1$, R′, R und A′ die obengenannten Bedeutungen haben, umsetzt oder Cephemverbindungen der Formel VI

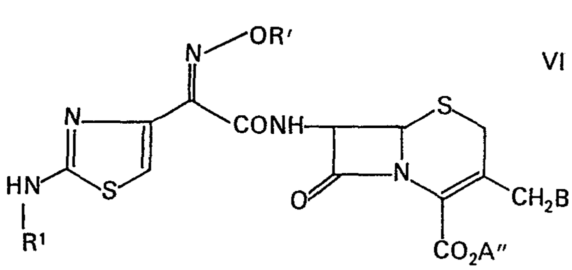

VI

in der $R^1$ und R′ die obengenannte Bedeutung besitzen, A″ für Wasserstoff oder ein Kation steht und B eine durch den nucleophilen Rest der Verbindung der Formel V austauschbare Gruppe darstellt mit einer Verbindung der allgemeinen Formel V

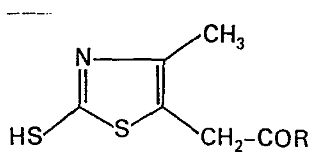

V

in der R die obengenannte Bedeutung hat, umsetzt und in den erhaltenen Verbindung gegebenenfalls

α) einen Rest $R^1$ und/oder A′ in der Bedeutung einer Schutzgruppe abspaltet und

ß) ein erhaltenes Salz direkt oder über die freien Carbonsäuren in einen physiologisch verträglichen Ester überführt.

In den vorstehenden Formeln steht

$R^1$ für Wasserstoff oder für eine aus der Peptidchemie bekannte Aminoschutzgruppe, wie z.B. gegebenenfalls substituiertes Alkyl mit 1 bis 6 C-Atomen, wie z.B. vorzugsweise tert.-Butyl, tert.-Amyl, Benzyl, 4-Methoxybenzyl, Benzhydryl, Trityl und Phenyläthyl, gegebenenfalls substituiertes aliphatisches Acyl mit 1 bis 4 C-Atomen, wie z.B. vorzugsweise Formyl, Chloracetyl, Bromacetyl, Trichloracetyl und Trifluoroacetyl oder gegebenenfalls substituiertes Alkoxycarbonyl mit 1 bis 4 C-Atomen im Alkylteil, wie beispielsweise Trichloräthoxycarbonyl oder Benzyloxycarbonyl,

A′ für ein Kation, eine Schutzgruppe oder eine physiologisch verträgliche Estergruppe, wie sie beispielhaft bereits unter A beschrieben wurden,

A″ für Wasserstoff oder ein Kation, beispielsweise ein solches, wie es bereits unter A erwähnt wurde und

B für eine durch den nucleophilen Rest der Verbindung der Formel V austauschbare Gruppe, insbesondere für Acyloxy mit 1 bis 4 C-Atomen, vorzugsweise Acetoxy, Halogen, vorzugsweise Chlor oder Brom, eine Azidogruppe, eine Carbamoyloxygruppe oder einen 2-Mercapto-pyridin-N-oxid-Rest.

Als reaktionsfähige Derivate der Carbonsäuren der allgemeinen Formel III eignen sich insbesondere die Halogenide, vorzugsweise Chloride und Bromide, ferner die Anhydride und gemischten Anhydride, die Azide und aktivierten Ester, vorzugsweise diejenigen mit p-Nitrophenol, 2,4-Dinitrophenol, Methylencyanhydrin, N-Hydroxysuccinimid und N-Hydroxyphthalimid, besonders bevorzugt diejenigen mit 1-Hydroxybenzotriazol und 6-Chlor-1-H-hydroxybenzotriazol.

Als gemischte Anhydride eignen sich besonders solche mit niederen Alkansäuren, z.B. mit Essigsäure und besonders bevorzugt mit substituierten Essigsäuren, wie z.B. Trichloressigsäure, Pivalinsäure oder Cyanessigsäure. Besonders geeignet sind aber auch die gemischten Anhydride mit Kohlensäurehalbestern, die man beispielsweise durch Umsetzung der Carbonsäure der allgemeinen Formel III, in denen $R^1$ nicht Wasserstoff bedeutet, mit Chlorameisensäurebenzylester, -p-nitrobenzylester, -isobutylester, -äthylester oder -allylester gewinnt.

Die Aktivierung kann auch durch Reaktion der Carbonsäuren der Formel III mit einem Reaktionsprodukt aus beispielsweise Phosgen, Thionylchlorid, Thionylbromid, Oxalylchlorid, Phosphorpentachlorid oder Phosphoroxychlorid mit einem N-dialkyl-substituierten Carbonsäureamid, wie z.B. Dimethylformamid, Dimethylacetamid oder N-Methylpyrrolidon erfolgen. Das Reaktionsprodukt aus Dimethylformamid und einem der obengenannten Halogenide wird in der Literatur auch als Vilsmeier-Reagens bezeichnet.

Die aktivierten Derivate können als isolierte Substanzen, aber auch in situ umgesetzt werden. Allgemein erfolgt die Umsetzung der Cephemderivate der Formel II mit einer Carbonsäure der Formel III oder einem aktivierten Derivat derselben in Gegenwart eines inerten Lösungsmittels. Insbesondere eignen sich chlorierte Kohlenwasserstoffe, wie vorzugsweise Methylenchlorid und Chloroform; Äther, wie z.B. Diäthyläther oder Diisopropyläther und vorzugsweise Tetrahydrofuran und Dioxan; Ketone, wie vorzugsweise Aceton und Butanon; Amide, wie vorzugsweise Dimethylformamid, Dimethylacetamid, N-Methylpyrrolidon oder Wasser. Es kann sich auch als vorteilhaft erweisen, Gemische der genannten Lösungsmittel zu verwenden. Dies ist oftmals der Fall, wenn die Cephemverbindung der Formel II mit einem in situ erzeugten aktivierten Derivat der Carbonsäure der Formel III umgesetzt wird.

Die Umsetzung von Cephemverbindungen der Formel II mit Carbonsäuren der Formel III bzw. deren aktivierten Derivaten kann in einem Temperaturbereich von etwa –50 bis etwa +80°C, vorzugsweise zwischen –20 und +50°C, besonders bevorzugt jedoch zwischen –20°C und Raumtemperatur erfolgen.

Die Reaktionsdauer hängt von den Reaktanten, der Temperatur und dem Lösungsmittel bzw. dem Lösungsmittelgemisch ab und liegt normalerweise zwischen etwa 1/4 und etwa 72 Stunden.

In einzelnen Fällen kann es sich auch als vorteilhaft erweisen, die freie Carbonsäure der Formel III mit einer Cephemverbindung der Formel II, in der A′ die Bedeutung eines leicht abspaltbaren Esterrestes als Schutzgruppe, wie vorzugsweise tert.-Butyl oder Trimethylsilyl hat, direkt umzusetzen, wobei man ein wasserbindendes Mittel in annähernd äquimolarer Menge, vorzugsweise in geringem Überschuss, zusetzt. Als solches kommen zum Beispiel Carbodiimide in Frage, insbesondere Dicyclohexylcarbodiimide. Diese Reaktion wird in inerten Lösungsmitteln, wie vorzugsweise Methylenchlorid, Dimethylformamid, Tetrahydrofuran, Dioxan oder auch Gemischen durchgeführt.

Die Umsetzung von aktivierten Derivaten der Carbonsäuren der Formel III mit Cephemverbindungen der Formel II wird vorzugsweise in alkalischem Milieu oberhalb pH 7, insbesondere zwischen pH-Werten von etwa 7 und etwa 9, vorgenommen. Man setzt hierfür dem Reaktionsgemisch eine Base zu, wie vorzugsweise Kalium, oder Natriumcarbonat, Kalium- oder Natriumbicarbonat, Kalium- oder Natriumhydroxid, Pyridin oder ein Trialkylamin, wie z.B. Triäthylamin, N-Methylmorpholin, Äthyldiisopropylamin oder Kalium-tert.- butylat.

Die Umsetzung kann vorzugsweise auch so durchgeführt werden, dass ein 1-Hydroxybenzotriazol- oder ein 6-Chlorhydroxybenzotriazolester

der Carbonsäuren der allgemeinen Formel III ohne Zusatz eines säurebindenden Mittels in einem Lösungsmittel direkt mit Cephemverbindungen der Formel II umgesetzt werden. Als Lösungsmittel haben sich insbesondere offenkettige und cyclische tertiäre Amide bewährt, besonders bevorzugt Dimethylformamid, Dimethylacetamid und N-Methylpyrolidon.

Die Abspaltung von $R^1$ kann mit in der ß-Lactam- und Peptid-Chemie allgemein üblichen schonenden Methoden, wie Hydrolyse in Säuren, vorzugsweise Ameisensäure oder Trifluoressigsäure, oder auch Hydrogenolyse in Gegenwart von Edelmetallkatalysatoren erfolgen. Je nach Schutzgruppe können aber auch spezielle Abspaltungsreagentien zum Einsatz kommen, wie beispielsweise gegebenenfalls substituierte Thioharnstoffe zum Entfernen von $\alpha$-Halogenacylgruppen.

Auch die Abspaltung einer Gruppe A', soweit sie die Funktion einer Schutzgruppe besitzt, kann mit in der ß-Lactam- und Peptid-Chemie üblichen schonenden hydrolytischen oder hydrogenolytischen Methoden herbeigeführt werden, wobei insbesondere Hydrolyse in anorganischen und organischen Säuren, wie vorzugsweise Trifluoressigsäure oder verdünnter Ameisensäure, genannt sein sollen.

Die Umsetzung von Verbindungen der Formel VI mit Verbindungen der Formel V wird vorzugsweise so ausgeführt, dass man ein Äquivalent einer Verbindung der allgemeinen Formel VI mit mindestens einem Äquivalent einer Verbindung der Formel V in einem Lösungsmittel umsetzt.

Ein bis zu etwa 10-facher Überschuss der Verbindung der Formel V wirkt sich vorteilhaft auf die Ausbeute aus. Der Überschuss beträgt vorzugsweise 1 bis 200%. Beispiele für die Reaktion nicht behindernde Lösungsmittel sind Wasser, Aceton, Butanon, Chloroform, Nitrobenzol, Methylenchlorid, Äthylenchlorid, Dimethylformamid, Dimethylacetamid, ß-Methylpyrrolidon, Methanol, Äthanol, Essigsäureäethylester, Tetrahydrofuran und Acetonitril, vorzugsweise Wasser.

Von den Lösungsmitteln können die hydrophilen Lösungsmittel, vorzugsweise Aceton, Methanol, Äthanol, Dimethylformamid und Acetonitril auch im Gemisch mit Wasser verwendet werden. Die Reaktion wird in einem pH-Bereich von etwa 5 bis etwa 8, vorzugsweise bei neutralem pH ausgeführt.

Bei der Reaktion können auch die Natrium- oder Kaliumsalze der Verbindungen der Formel V eingesetzt werden.

Die Reaktionstemperatur kann in einem weiten Bereich variiert werden. In der Regel wird die Reaktion bei etwa 50 bis etwa 80°C, vorzugsweise bei etwa 65°C, durchgeführt.

Die obengenannten Verbindungen der Formel V, in denen R Alkoxy, Alkenoxy, Alkinoxy, Phenyloxy oder Phenylalkoxy bedeutet, die in der oben angegebenen Weise substituiert sein können, lassen sich herstellen, indem man die 2-Mercapto-4-methyl-1,3-thiazolessigsäure mit einem Alkohol ROH, wobei R die oben angegebene Bedeutung hat, mit oder ohne Lösungsmittel in Gegenwart eines Katalysators, wie beispielsweise Salzsäure, in literaturbekannter Weise umsetzt. Es kann auch die Thiazolessigsäure mit den Alkoholen unter Zugabe von Carbodiimiden in einem inerten Lösungsmittel in literaturbekannter Weise umgesetzt werden.

Die Verbindung der Formel V kann auch durch Ringschluss aufgebaut werden. Hierbei wird zunächst der ß-Halogen-lävulinsäureester durch Veresterung der Säure mit einem Alkohol ROH, wobei R die oben angegebene Bedeutung hat, in literaturbekannter Weise hergestellt. Den ß-Halogen-lävulinsäureester kann man mit Ammoniumdithiocarbamat in wässrig-alkoholischer Lösung, wobei das Mischungsverhältnis in weiten Grenzen schwanken kann, bei 0 bis 30°C, vorzugsweise bei 5 bis 10°C, zum Thiazol umsetzen.

Die obengenannten Verbindungen der Formel V, in denen R für eine der angegebenen, gegebenenfalls substituierten Aminogruppen steht, kann man z.B. herstellen, indem man eine Verbindung der Formel V, in der R eine Alkyl-O-Gruppe mit 1 bis 4 C-Atomen, vorzugsweise Methoxy und Äthoxy sein kann, mit einem Amin, das in der angegebenen Weise substituiert sein kann, in einem inerten Lösungsmittel bei erhöhten Temperaturen von etwa 40 bis etwa 100°C, vorzugsweise 50 bis 60°C, umsetzt.

Ausserdem können Verbindungen der Formel V, in denen R für eine der angegebenen, gegebenenfalls substituierten Aminogruppen steht, auch dadurch erhalten werden, dass man ß-Halogenlävulinsäuren, in denen Halogen für Chlor, Brom oder Jod stehen kann, mit den Aminen in Gegenwart eines wasserentziehenden Mittels, wie beispielsweise von Carbodiimiden, vorzugsweise eines Dicyclohexylcarbodiimids, in einem aprotischen Lösungsmittel bei erhöhter Temperatur von etwa 30 bis etwa 100°C, vorzugsweise 60 bis 70°C, zu den ß-Halogenamiden umsetzt.

Die Amide werden dann mit Ammoniumdithiocarbamat in wässrig-alkoholischer Lösung, wobei das Mischungsverhältnis in weiten Grenzen schwanken kann, bei etwa 0 bis 30°C, vorzugsweise bei 5 bis 10°C, zum Thiazol umgesetzt.

Die Ausgangsverbindungen der Formeln III und VI sind literaturbekannt oder können nach literaturbekannten Verfahren erhalten werden.

Ausgangsverbindungen der Formel II können in derselben Weise erhalten werden, wie sie für die Umsetzung der Verbindungen der Formel V mit Verbindungen der Formel VI beschrieben wurde.

Die erfindungsgemäss erhaltenen Verbindungen der Formel I zeigen bemerkenswert gute antibakterielle Wirksamkeiten sowohl gegen grampositive, als auch gramnegative bakterielle Keime.

Auch gegen penicillinase- und cephalosporinasebildende Bakterien sind die neuen Verbindungen unerwartet gut wirksam. Da sie zudem günstige toxikologische und pharmakologische Eigenschaften zeigen, stellen sie wertvolle Chemotherapeutika dar.

Die Erfindung betrifft somit auch Arzneipräparate zur Behandlung von mikrobiellen Infektionen, die durch einen Gehalt an einer oder mehreren der erfindungsgemässen Verbindungen charakterisiert sind.

Die erfindungsgemässen Produkte können auch in Kombinationen mit anderen Wirkstoffen, beispielsweise aus der Reihe der Penicilline, Cephalosporine oder Aminoglykoside zur Anwendung kommen.

Die Verbindungen der Formel I können oral, intramuskulär oder intravenös verabfolgt werden.

Arzneipräparate, die eine oder mehrere Verbindungen der allgemeinen Formel I als Wirkstoff enthalten, können hergestellt werden, indem man die Verbindungen der allgemeinen Formel I mit einem oder mehreren pharmakologisch verträglichen Trägerstoffen oder Verdünnungsmitteln, wie z.B. Füllstoffen, Emulgatoren, Gleitstoffen, Geschmackskorrigentien, Farbstoffen oder Puffersubstanzen vermischt und in eine geeignete galenische Zubereitungsform bringt, wie beispielsweise Tabletten, Dragees, Kapseln, oder eine zur parenteralen Applikation geeignete Lösung oder Suspension. Als Träger- oder Verdünnungsmittel seien beispielsweise erwähnt Traganth, Milchzucker, Agar-Agar, Polyglykole, Talkum, Äthanol und Wasser. Für die parenterale Application kommen vorzugsweise Suspensionen oder Lösungen in Wasser in Betracht. Es ist auch möglich, die Wirkstoffe als solche ohne Träger- oder Verdünnungsmittel in geeigneter Form beispielsweise in Kapseln zu applizieren.

Geeignete Dosen der Verbindungen der allgemeinen Formel I liegen bei etwa 0,4 bis 20 g/Tag, vorzugsweise bei 0,5 bis 4 g/Tag für einen Erwachsenen von etwa 60 kg Körpergewicht. Es können Einzel- oder im allgemeinen Mehrfachdosen verabfolgt werden, wobei die Einzeldosis den Wirkstoff in einer Menge von etwa 50 bis 1000 mg, vorzugsweise 100 bis 500 mg enthalten kann.

Gegenüber ähnlichen, aus der belgischen Patentschrift Nr. 865 632 bekannten Cephemderivaten zeichnen sich die erfindungsgemässen Verbindungen durch unerwartete überlegene antibakterielle und pharmakokinetische Eigenschaften aus.

Erfindungsgemäss lassen sich ausser den in den Ausführungsbeispielen genannten Verbindungen beispielsweise die in den folgenden Tabellen genannten Verbindungen I darstellen:

Tabelle 1

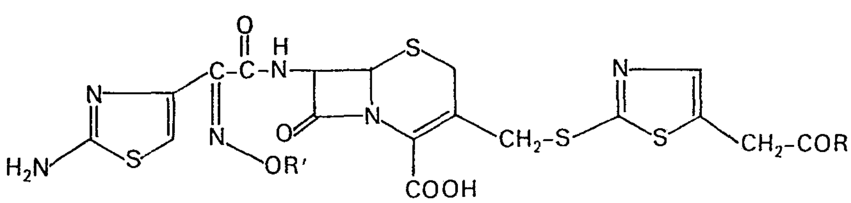

| R' | R |
|---|---|
| $-CH_3$ | $-O-(CH_2)_3-CH_3$ |
| $-CH_3$ | $-O-CH=CH_2$ |
| $-CH_3$ | -O-CH=CH- 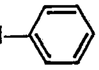 |
| $-CH_3$ | $-O-CH_2-O-CH_3$ |
| $-CH_3$ | $-O-C\equiv CH$ |
| $-CH_3$ | $-O-C-CH_3$<br>‖<br>$CH_2$ |

Die Beispiele erläutern die Erfindung, ohne sie jedoch einzuschränken.

Beispiel 1:

7-Beta-[alpha-syn-methoximino-alpha-(2-aminothiazol-4-yl)acetamido]-3-(4-methyl-5-p-methoxybenzyl-oxycarbonylmethyl-1,3-thiazol-2-yl-thiomethyl)-3-cephem-4-carbonsäure

760 mg α-syn-Methoximino-α-(2-amino-thiazol-4-yl)-essigsäure werden in 20 ml trockenem Dimethylacetamid gelöst und mit 530 mg Hydroxybenztriazol und 790 mg Dicyclohexylcarbodiimid versetzt. Die gelbe Lösung wird 2 Stunden gerührt und von entstandenem Harnstoff abgesaugt. Die verbleibende Lösung wird mit einer Lösung von 2 g 7-Amino-3-(4-methyl-5-p-methoxybenzyloxycarbonylmethyl-1,3-thiazol-2-yl-thiomethyl)-3-cephem-4-carbonsäure in 10 ml Dimethylacetamid versetzt.

Es wird bei Raumtemperatur über Nacht gerührt und langsam mit Wasser versetzt. Der Niederschlag wird abgesaugt und mit Wasser gereinigt.

I.R.: 3318, 2940, 1780, 1730, 1675 $cm^{-1}$

NMR (DMSO) δ ppm: 2,13 s 3H, $CH_3$; 5,0 s 2H, $2CH_2$, 5,63 m 1H 7H; 6,66 s 1H 5H syn; 7,0 m 4H arom.; 9,43 d. 1H 7NH

Herstellung der
7-Amino-3-(4-methyl-5-p-methoxybenzyloxycarbonylmethyl-1,3-thiazol-2-yl-thiomethyl)-3-cephem-4-carbonsäure

2,72 g 7-Aminocephalosporansäure werden mit 20 ml Wasser und 10 ml Aceton versetzt. Nach Zugabe von 1,68 g Natriumhydrogencarbonat in 20 ml Wasser werden 3,03 g 2-Mercapto-4-methyl-5-(p-methoxybenzyl-oxycarbonylmethyl)1,3-thiazol zugesetzt. Ein pH von 6,5 wird mit 2n HCl eingestellt und auf 65°C hochgeheizt. Nach 6 Stunden und Stehen über Nacht wird am Rotavapor das Aceton eingedampft und der Rückstand mit Essigester extrahiert. Nach dem Ansäuern auf pH 2,8 wird abgesaugt.
NMR (DMSO) α ppm: 2,33 s 3H $CH_3$ thiazol

Beispiel 2:

Analog Beispiel 1 wird 7-Beta-[alpha-syn-methoximino-alpha-(2-aminothiazol-4-yl)acetamido]-3-(4-methylbenzyloxycarbonylmethyl-1,3-thiazol-2-yl-thiomethyl)-3-cephem-4-carbonsäure hergestellt.
I.R.: 1773, 1735, 1632
NMR (DMSO) δ ppm: 5,1 s 2H $CH_2$; 5,53 q 1H 7H; 6,7 s 1H 5H; 7,3 s 5H arom.

Beispiel 3:

7-Beta-[alpha-syn-methoximino-alpha-(2-aminothiazol-4-yl)acetamido]-3-(4-methyl-5-p-methoxybenzyloxycarbonylmethyl-1,3-thiazol-2-ylthiomethyl)-3-cephem-4-carbonsäure.

9,5 g Natriumsalz der 7-Beta [alpha-syn-methoximino-alpha(2-amino-thiazol-4-yl)-acetamido]-cephalosporansäure werden in 150 ml Wasser gelöst und mit 3 ml gesättigter Natriumhydrogencarbonatlösung auf pH 7,2 gestellt. Danach werden 9,0 g 2-Mercapto-4-methyl-5-(p-methoxybenzyl-oxycarbonylmethyl)-1,3-thiazol in 150 ml Aceton gelöst zugegeben.

Bei 65° wird 12 Stunden gerührt. Durch stufenweise Zugabe von Natriumhydrogencarbonatlösung wird der pH auf 6,0 gehalten. Nach dem Erkalten wird das Aceton im Vakuum eingedampft und der Rückstand mehrmals mit Essigester extrahiert. Die wässrige Phase wird etwas eingeengt, um den Essigester zu vertreiben und dann unter guter Kühlung auf pH 2,8 mit verdünnter Salzsäure angesäuert. Der entstandene Niederschlag wird abgesaugt und getrocknet.
I.R.: 3318, 2940, 1780, 1730, 1675 $cm^{-1}$
NMR (DMSO) δ ppm: 2,13 s 3H $CH_3$; 5,0 s 2H $CH_2$; 5,63 q 1H 7H; 6,66 s 1H 5H syn; 7,0 m 4H arom.; 9,43 d 1H 7NH

Beispiel 4:

Analog dem in Beispiel 3 beschriebenen Verfahren gewinnt man 7-Beta-[alpha-syn-methoximino-alpha(2-amino-thiazol-4-yl)acetamido]-3-(4-methyl-5-benzyloxycarbonylmethyl-1,3-thiazol-2-yl-thiomethyl)-3-cephem-4-carbonsäure.
I.R.: 3315, 2940, 1780, 1734, 1675 $cm^{-1}$
NMR (DMSO) δ ppm: 2,13 s 3H $CH_3$; 5,0 s 2H $CH_2$; 5,63 q 1H 7H; 6,66 s 1H 5H syn; 7,0 m 4H arom.; 9,43 d 1H 7NH

Beispiel 5:

7-Beta-[alpha-syn-methoximino-alpha(2-amino-thiazol-4-yl)acetamido]-3-[4-methyl-5-(3,4-dimethoxyphenyl oxycarbonylmethyl)-1,3-thiazol-2-yl-thiomethyl]-3-cephem-4-carbon-säure (analog Beispiel 3 erhalten)
I.R.: 1775, 1662, 1520 $cm^{-1}$
NMR (DMSO) δ ppm: 2,2 s 3H $CH_3$; 5,03 d. 1H; 3,7 s 6H $OCH_3$; 5,7 q 1H 7H 6,66 s 1H 5H syn; 6,86 s 2H arom. 9,5 d. 1H 7H

Beispiel 6:

7-Beta-[alpha-syn-methoximino-alpha(2-aminothiazol-4-yl)acetamido]-3-(4-methyl-5-p-chlorphenyl-oxycarbonylmethyl-1,3-thiazol-2-yl-thiomethyl)-3-cephem-4-carbonsäure.
(analog Beispiel 3 erhalten)
I.R.: 1775 $cm^{-1}$, 1630 $cm^{-1}$
NMR (DMSO) δ ppm: 2,2 ppm s 3H thiazol $CH_3$; 3,83 $CH_2$ 3,1 s 2H $CH_2$; 5,7 q. 1H 7H; 6,73 s syn 5H thiazol, 7,36 s 3H arom; 9,56 d. 1H NH

Beispiel 7:

7-Beta-[alpha-syn-methoximino-alpha(2-amino-thiazol-4-yl)acetamido]-3-(4-methyl-5-cinnamyl-oxycarbonyl-methyl-1,3-thiazol-2-yl-thiomethyl)-3-cephem-4-carbonsäure
(analog Beispiel 3 erhalten)
I.R.: 1775, 1726, 1670 $cm^{-1}$
NMR (DMSO) ppm: 2,33 s 3H $CH_3$; 3,8 s 2H $CH_2$; 4,73 d. 2H $CH_2$=; 5,1 d 1H 6H; 5,73 q. 1H 7H; 6; 5 d 2H CH=CH; 6,7 s 1H 5H thiazol; 7,36 m 5H; 9,53 d. 1H 7NH

Beispiel 8:

7-Beta-[alpha-syn-methoximino-alpha(2-amino-thiazol-4-yl)acetamido]-3-(4-methyl-5-p-methoxyphenyl-oxycarbonylmethyl-1,3-thiazol-2-ylthiomethyl)-3-cephem-4-carbonsäure.
(analog Beispiel 3 erhalten)

Beispiel 9:

7-Beta-[alpha-syn-methoximino-alpha(2-aminothiazol-4-yl)acetamido]-3-(4-methyl-5-beta-phenyläthyloxy-carbonylmethyl-1,3-thiazol-2-yl-thiomethyl)-3-cephem-4-carbonsäure
(analog Beispiel 3 erhalten)
I.R.: 1770, 1640 $cm^{-1}$
NMR (DMSO) δ ppm: 2,2 ppm s 3H $CH_3$ thiazol; 3,8 s 4H $CH_2$; 5,1 d. 1H 6H; 5,7 q. 1H 7H; 6,7 s 1H 5H thiazol 7, 13 s 5H 9,5 d. 1H NH

Beispiel 10:

7-Beta-[alpha-syn-methoximino-alpha(2-amino-thiazol-4-yl acetamido]-3-(4-methyl-5-alpha-phenyläthyloxy carbonylmethyl-1,3-thiazol-2-yl-thiomethyl)-3-cephem-4-carbonsäure
(analog Beispiel 3 erhalten)
I.R.: 1773, 1630
NMR (DMSO) δ ppm: 2,23 s 3H $CH_3$ thiazol; 3,9 s 2H $CH_2$ 16,8 s 1H 5H; 7,3 s 5H arom. 9,5 d. 1H 7NH

Beispiel 11:

7-Beta-[alpha-syn-methoximino-alpha(2-amino-thiazol-4-yl)acetamido]-3-(4-methyl-5-beta-

phenoxy-äthyl-oxy-carbonylmethyl-1,3-thiazol-2-yl-thiomethyl)-3-cephem-4-säure
(analog Beispiel 3 erhalten)
NMR (DMSO) δ ppm: 2,23 s 3H $CH_3$-thiazol 3,9 s 2H $CH_2$; 5,83 q. 1H 7H; 6,8 s 1H 5H 7,3 s 5H m; 9,5 d. 1H 7NH

Beispiel 12:
7-Beta-[alpha-syn-methoximino-alpha(2-amino-thiazol-4-yl acetamido]-3-(4-methyl-5-isobutyloxycarbonylmethyl-1,3-thiazol-2-yl-thiomethyl)-3-cephem-4-carbonsäure
(analog Beispiel 3 erhalten)
I.R.: 1780, 1725, 1672
NMR (DMSO) δ ppm: 0,8 s 3H; 0.9 s 3H $CH_3$; 2,2 s 3H $CH_3$ 3,86 s 2H $CH_2$; 5,06 d. 1H 6H; 5,7 q. 1H 7H; 6,66 s 1H thiazol 5H; 9,5 d. 7NH

Beispiel 13:
7-Beta-[alpha-syn-methoximino-alpha(2-amino-thiazol-4-yl)acetamido]-3-(4-methyl-5-tert.-butyl-oxycarbonyl-methyl-1,3-thiazol-2-yl-thiomethyl)-3-cephem-4-säure
(analog Beispiel 3 erhalten)
I.R.: 1760, 1638
NMR (DMSO) δ ppm: 1,4 s 9H -$(CH_3)_3$; 2,2 s 3H thiazol $CH_3$; 5,1 d 6H, 5,73 q. 1H; 6,7 s 1H, 5H; 9,5 d 1H NH

Beispiel 14:
7-Beta-[alpha-syn-methoximino-alpha(2-amino-thiazol-4-yl)acetamido]-3-(4-methyl-5-allyl-oxycarbonylmethyl-1,3-thiazol-2-yl-thiomethyl)-3-cephem-4-carbonsäure
(analog Beispiel 3 erhalten)
I.R.: 1770, 1730, 1660
NMR (DMSO) δ ppm: 2,2 s 3H $CH_3$ thiazol; 3,9 s 2H $CH_2$; 4,6 s 2H $CH_2$; 5, 13

Beispiel 15:
7-Beta-[alpha-syn-methoximino-alpha(2-amino-thiazol-4-yl)acetamido]-3-[4-methyl-5-(2-propinyloxycarbonyl-methyl)-1,3-thiazol-2-yl-thiomethyl]-3-cephem-4-carbonsäure
(analog Beispiel 3 erhalten)
I.R.: 1778, 1680 $cm^{-1}$
NMR (DMSO) δ ppm: 2,2 s 3H $CH_3$ thiazol; 3,8 s 2H $CH_2$; 4,7 d 2H $CH_2$ 5,02 d 1H 6H; 5,7 q. 1H 7H; 6,66 s 1H 5H thiazol; 9,49 d 1H 7NH

Beispiel 16:
7-Beta-[alpha-syn-methoximino-alpha(2-amino-thiazol-4-yl)acetamido]-3-(4-methyl-5-carbamoylmethyl-1,3-thiazol-2-yl-thiomethyl)-3-cephem-4-carbonsäure
(analog Beispiel 3 erhalten)
I.R.: 1770, 1670
NMR (DMSO) δ ppm: 2,2 s 3H $CH_3$ thiazol; 3,76 s 2H $CH_2$; 3,03 d 1H 6H; 5,66 q. 1H 7H; 6,66 s 1H 5H 9,49 d. 1H NH

Beispiel 17:
7-Beta-[alpha-syn-methoximino-alpha(2-amino-thiazol-4-yl)acetamido]-3-(4-methyl-5-N-phenyl-carbamoyl-methyl-1,3-thiazol-2-yl-thiomethyl)-3-cephem-4-carbonsäure
(analog Beispiel 3 erhalten)
I.R.: 1775, 1640
NMR (DMSO) δ ppm: 2,26 s 3H $CH_3$; 5,03 d 1H 6H; 5,63 q. 1H 7H; 6,66 ppm s 1H thiazol; 7,33 m 4H arom.; 9,5 d. 1H 7H

Beispiel 18:
7-Beta-[alpha-syn-methoximino-alpha(2-amino-thiazol-4-yl)acetamido]-3-(4-methyl-5-N-methylcarbamoyl-methyl-1,3-thiazol-2-yl-thiomethyl)-3-cephem-4-carbonsäure
(analog Beispiel 3 erhalten)

Beispiel 19:
7-Beta-[alpha-syn-methoximino-alpha(2-amino-thiazol-4-yl)acetamido]-3-(4-methyl-5-beta-methoxyäthoxycarbonylmethyl-1,3-thiazol-2-yl-thiomethyl)-3-cephem-4-carbonsäure
NMR ($D_2O$): 2,2 s 3H $CH_3$ thiazol, 3,3 s 3H $OCH_3$, 3,93 s 3H $NOCH_3$; 3,65 s 2H $CH_2$; 5,1 d 1H 6H; 5,7 d 1H 7H; 6,9 s 1H thiazol
(analog Beispiel 3 erhalten)

Beispiel 20:
7-Beta-[alpha-syn-methoximino-alpha(2-amino-thiazol-4-yl)acetamido]-3-(4-methyl-5-äthyl-oxycarbonyl-methyl-1,3-thiazol-2-yl-thiomethyl)-3-cephem-4-carbonsäure
(analog Beispiel 3 erhalten)
I.R.: = 1774 $cm^{-1}$ = 1723 $cm^{-1}$
NMR (DMSO) δ ppm: 1,18 p • t 3H, 2,18 3H; 3,79 s 4,07 q, 4,22 q, 5,05 d. 1H; 5,70 q 1H; 6,70 s 1H 7,11 2H; 9,48 d. 1H

Beispiel 21:
7-Beta-[alpha-syn-methoximino-alpha-2-amino-thiazol-4-yl)acetamido]-3-(4-methyl-5-methyloxy-carbonylmethyl-1,3-thiazol-2-yl-thiomethyl)-3-cephem-4-carbonsäure
(analog Beispiel 3 erhalten)
I.R.: 1775 $cm^{-1}$, 1730 $cm^{-1}$, 1680 $cm^{-1}$
NMR (DMSO) δ: 2,23 s 3H; 3,64 s 3H; 3,80 s $CH_2$ oxim; 3,9 s 2H; 4,25 q. $CH_2$; 5,08 d 6H; 5,73 dd 1H; 6,70 s 1H 7,13 s 9,53 d

Analyse

| | | | | |
|---|---|---|---|---|
| Ber.: | C 41,0 | H 3,9 | N 13,6 | S 20,8 |
| Gef.: | C 41,0 | H 3,9 | N 13,5 | S 20,0 |

Herstellung von Ausgangsverbindungen

Beispiel A:
2-Mercapto-4-methyl-5-methoxycarbonyl-1,3-thiazol

12,0 g α-(2-Mercapto-4-methyl)1,3-thiazolessigsäure werden in 100 ml Methanol gelöst und mit 2,0 ml konz. Salzsäure versetzt. Bei Zimmertemperatur wird 4 Stunden gerührt. Danach wird im Vakuum eingeengt. Der Rückstand wird mit Essigester versetzt und mehrmals mit Natriumhydrogencarbonatlösung gewaschen. Nach dem Trocknen der vereinigten Essigesterextrakte über

MgSO4 wird im Vakuum vollständig eingeengt.
I.R.: 1635, 1748 $cm^{-1}$
NMR (DMSO) δ ppm: 2,03 ppm s 3H, 3,26 s 2H; 3,6 s 3H

Beispiel B:
2-Mercapto-4-methyl-5-(p-methoxybenzyloxycarbonylmethyl)-1,3-thiazol

10,8 g α-(2-Mercapto-4-methyl)-1,3-thiazolessigsäure werden in 100 ml Methylenchlorid und 15 ml Dimethylformamid gelöst. Nach Zugabe von 570 mg Dimethylaminopyridin und 8,1 g Anisalkohol werden unter Eiskühlung 13,8 g Dicyclohexylcarbodiimid in 50 ml Methylenchlorid eingetropft. Das Gemisch erwärmt sich leicht und wird 1,5 Stunden bei Zimmertemperatur gerührt. Von ausgefallenem Harnstoff wird abgesaugt und am Rotationsverdampfer bei gutem Vakuum eingedampft, in Essigester gelöst und mit Natriumhydrogencarbonatlösung gewaschen. Der Essigesterextrakt wird mit Natriumsulfat getrocknet und eingeengt. Es wird mit Äther angerieben und aus Methanol umkristallisiert.
I.R.: 1748, 1630, 1620 $cm^{-1}$
NMR (DMSO) δ ppm = 2,06 ppm s 3H thiazol $CH_3$,

3,7 s 2H, 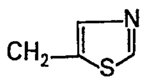 3,76 s 3H $OCH_3$; 5,03 s 2H

$CH_2$–O; 6,75 - 7,33 m 4H arom. H

Analyse

| | | | |
|---|---|---|---|
| Ber. | C 54,4 | H 4,9 | N 4,8 |
| Gef. | C 54,0 | H 5,1 | N 4,9 |

Analog Beispiel B wurden hergestellt:

C) 2-Mercapto-4-methyl-5-(benzyl-oxycarbonyl-methyl)-1,3-thiazol
NMR (DMSO) δ ppm = 2,05 ppm s 3H thiazol $CH_3$;

3,61 ppm s 2H 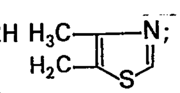 5,06 ppm s 2H

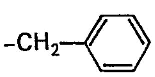 ; 7,2 ppm s 5H arom.

D) 2-Mercapto-4-methyl-5-(3,4-dimethoxybenzyloxycarbonylmethyl)1,3-thiazol
I.R.: 1730, 1637, 1603 $cm^{-1}$
NMR (DMSO): 2,03 ppm s 3H $CH_3$; 3,23 s 2H $CH_2$ δ ppm: 3,7 ppm s 6H $OCH_3$; 5,0 s 2H $CH_2$–O; 6,86 m 3H arom.

E) 2-Mercapto-4-methyl-5-(p-chlorbenzyloxycarbonyl-methyl)-1,3-thiazol
I.R.: 1730 16 30 $cm^{-1}$

F) 2-Mercapto-4-methyl-5-(cinnamyloxycarbonylmethyl)-1,3-thiazol
I.R.: 1726, 1625 $cm^{-1}$
NMR (DMSO) δ ppm: 2,06 ppm s 3H $CH_3$; 3,7 ppm s 2H $CH_2$ 4,7 ppm d 2H = $CH_2$; 6,43 ppm q 1H CH=7,33 ppm m 5H

G) 2-Mercapto-4-methyl-5-(p-methoxyphenyl-oxycarbonyl)-1,3-thiazol
NMR (DMSO) δ ppm: 6,9 m 4H arom.

H) 2-Mercapto-4-methyl-5-(alpha-phenyläthyloxycarbonylmethyl)1,3-thiazol
NMR (DMSO) δ ppm: 2,03 s 3H Thiazol; 1,4 s 3H

$CH_3$; 3,7 s 2H $CH_2$; 5,8 q 1,H 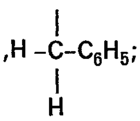

7,3 m. aromat. H

I) 2-Mercapto-4-methyl-5-(beta-phenyläthyloxycarbonylmethyl)1,3-thiazol
NMR (DMSO) δ ppm: 2,0 s 3H thiazol; 3,6 2H $CH_2$; 4,26 tripl $CH_2$; 7,16 m 5H arom.

J) 2-Mercapto-4-methyl-5-(alpha-phenoxyäthyl-oxycarbonyl-methyl)-1,3-thiazol

K) 2-Mercapto-4-methyl-5-(isobutyloxycarbonylmethyl)-1,3-thiazol

NMR (DMSO) δ ppm: 0,86 d 6H 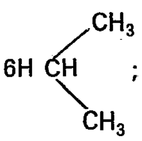 ; 2,06 s 3H.

thiazol $CH_3$, 2,76 ppm d 2H $CH_2$, 3,8 d 2H $CH_2$

L) 2-Mercapto-4-methyl-5-(tert.-butyloxycarbo-
nylmethyl)-1,3-thiazol
NMR (DMSO) δ ppm: 1,4 s 9H 3 $CH_3$; 2,1,3H $CH_3$, 3,93 2H $CH_2$

M) 2-Mercapto-4-methyl-5-(allyl-oxycarbonylmethyl)-1,3-thiazol
NMR (DMSO) δ ppm: 2,03 s 3H thiazol $CH_3$; 3,63 s 2H $CH_2$; 4,53 d 2H $CH_2$; 5,23 m CH; $CH_2$=; 5,87 m; 1H CH=

N) 2-Mercapto-4-methyl-5-(2-propinyl-oxycarbonylmethyl)-1,3-thiazol
NMR (DMSO) δ ppm: 2,03 s 3H $CH_3$ thiazol; 2,56 1H q. 3,7 s 2H $CH_2$; 4,7 d 2H

O) 2-Mercapto-4-methyl-5-(betamethoxyäthyl-oxycarbonyl-methyl)-1,3-thiazol
I.R.: $cm^{-1}$ 3593, 2940, 2720, 1740, 1650, 1630, 1470, 1450
NMR (DMSO) δ ppm: 2,1 s 3H $CH_3$; 3,25 s 3H $OCH_3$ 3,4 - 3,4 s 2H; 4,3,; 7,95

P) 2-Mercapto-4-methyl-5-(carbamoylmethyl)-1,3-thiazol

20,0 g α-(2-Mercapto-4-methyl)-1,3-thiazolessigsäuremethylester werden in einem Autoklaven mit 40 ml Wasser versetzt und unter Kühlung mit 100 ml flüssigem Ammoniak zugegeben. Im Autoklaven wird bei Raumtemperatur 22 Std. unter Stickstoff geschüttelt. Nach dem Abblasen des Ammoniaks mit Stickstoff verbleibt eine gelbe Substanz. Diese wird mit Wasser versetzt und

mit verdünnter Salzsäure auf pH 1 gestellt. Der Niederschlag wird abgesaugt, mit Wasser gewaschen und getrocknet.
I.R.: 1650–80, 1595 $cm^{-1}$
NMR (DMSO) δ ppm: 2,03 s 3H; 3,3 s 2H

Analyse $C_6H_8N_2S_2O$

| | | | | |
|---|---|---|---|---|
| Ber.: | C 38,4% | H 4,2% | N 15,0% | S 34,1% |
| Gef.: | C 37,7% | H 4,1% | N 14,5% | S 35,0% |

Q) 2-Mercapto-4-methyl-5-(N-methylcarbamoylmethyl)-1,3-thiazol
NMR (DMSO) δ ppm: 3,36 s 3H NH $CH_3$; 7,86 ber. s NH; 2,06 s 3H; 2,72 s 2H

R) 2-Mercapto-4-methyl-5-(N-phenylcarbamoylmethyl)-1,3-thiazol

18,0 g β-Bromlävulinsäureanilid werden in 50,0 ml Methanol gelöst und 8,0 g frisch hergestelltes Ammoniumditbiocarbamat gelöst in 10 ml Wasser bei 0° zugetropft. Die Temperatur steigt von 0° auf 10°C an. Es wird einen Tag bei Raumtemperatur nachgerührt, von Ammoniumbromid abgesaugt und das Filtrat am Rotavapor eingeengt. Der Rückstand wird aus einem Methanol-Wasser-Gemisch umkristallisiert.
I.R.: 3270, 3024, 2862, 1656, 1600, 1528 $cm^{-1}$
NMR (DMSO) δ ppm: 2,2 s 3H $CH_3$ thiazol; 3,62 s $CH_2$ 2H; 7,4 m 5H arom.

Herstellung von β-Bromlävulinsäureanilid

5,0 g Anilin, 9,5 g β-Bromlävulinsäure und 10,0 g Dicyclohexylcarbodiimid werden in jeweils 25 ml Tetrahydrofuran gelöst. Die Lösung des Carbodiimids wird zur Lösung des Anilins und der β-Bromlävulinsäure unter Kühlung bei 0° eingetropft. Es bildet sich sofort ein Niederschlag. Der Dicyclohexylharnstoff wird nach 24 Stunden abgesaugt und das Filtrat im Rotavapor eingeengt. Das verbliebene Öl wird in Essigester gelöst mit Natriumhydrogencarbonatlösung und verdünnter Salzsäure gewaschen. Nach dem Trocknen und Eindampfen des Lösungsmittels verbleibt ein hellbraunes Öl.
I.R.: 3230, 2935, 2845, 1710, 1600 $cm^{-1}$
NMR (DMSO) δ ppm: 2,33 d 3H $CH_3$; 3,0 q 2H $CH_2$; 4,86 q 1H CH; 7,3 m 5H arom.

**Patentansprüche**

1. Cephemderivate der allgemeinen Formel I

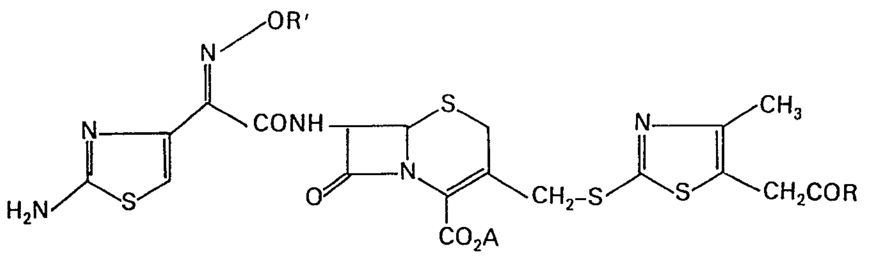

I

in der R die Bedeutung besitzt
von Alkoxy mit 1–4 C-Atomen, das substituiert sein kann durch Alkoxy mit 1–4 C-Atomen oder Phenyloxy, von Alkenoxy mit 2–3 C-Atomen, das substituiert sein kann durch Phenyl,
von Alkinoxy mit 2–3 C-Atomen,
von Phenyloxy, das substituiert sein kann durch Halogen, Alkyl mit 1–4 C-Atomen oder Alkoxy mit 1–4 C-Atomen,
von Phenylalkoxy mit 1–2 C-Atomen im Alkylteil, wobei der Phenylteil substituiert sein kann durch Halogen, Alkyl mit 1–4 C-Atomen oder Alkoxy mit 1–4 C-Atomen,
von Amino,
von Alkyl- oder Dialkylamino mit 1–4 C-Atomen pro Alkylteil,
von Phenylamino, wobei der Phenylteil substituiert sein kann durch Halogen, Alkyl mit 1–4 C-Atomen oder Alkoxy mit 1–4 C-Atomen,
R′ steht für Alkyl mit 1–4 C-Atomen und
A für Wasserstoff, ein physiologisch unbedenkliches Kation oder eine physiologisch unbedenkliche Estergruppe und in der die R′O-Gruppe in syn-Position steht.

2. Verfahren zur Herstellung von Cephemderivaten der allgemeinen Formel I, dadurch gekennzeichnet, dass man Lactame der allgemeinen Formel II

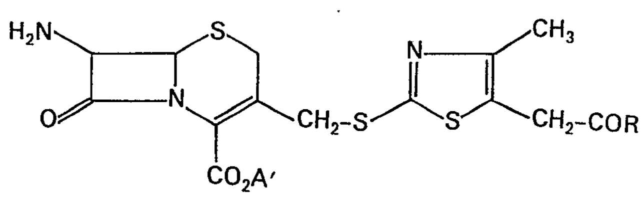

II

in der R die oben angegebenen Bedeutungen hat und A′ für ein Kation, eine Schutzgruppe oder einen physiologisch unbedenklichen Ester steht, mit einer Carbonsäure der allgemeinen Formel III

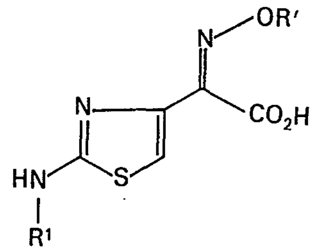

III

oder einem reaktionsfähigen Derivat derselben, in der $R^1$ für Wasserstoff oder eine aus der Peptidchemie bekannte Aminoschutzgruppe steht und $R'$ die obige Bedeutung besitzt, zu einer Verbindung der allgemeinen Formel IV

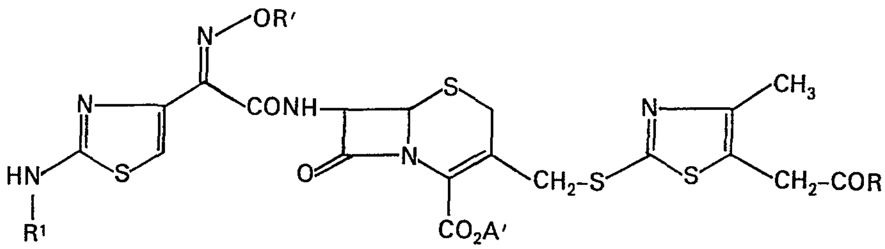

IV

in der $R^1$, $R'$, R und $A'$ die obengenannten Bedeutungen haben, umsetzt oder Cephemverbindung der Formel VI

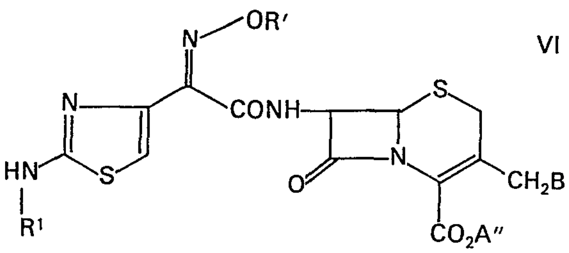

VI

in der $R^1$ und $R'$ die obengenannte Bedeutung besitzen, $A''$ für Wasserstoff oder ein Kation steht und B eine durch den nucleophilen Rest der Verbindung der Formel V austauschbare Gruppe darstellt, mit einer Verbindung der allgemeinen Formel V

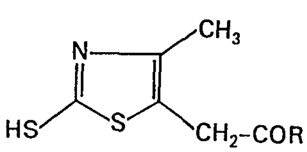

V

in der R die obengenannte Bedeutung hat, umsetzt und in den erhaltenen Verbindungen gegebenenfalls

α) einen Rest $R^1$ und/oder $A'$ in der Bedeutung einer Schutzgruppe abspaltet und

β) ein erhaltenes Salz direkt oder über die freie Carbonsäure in einen physiologisch unbedenklichen Ester überführt.

3. Gegen bakterielle Infektionen wirksame pharmazeutische Präparate, gekennzeichnet durch einen Gehalt an Cephemderivaten der allgemeinen Formel I.

4. Verfahren zur Herstellung von gegen bakterielle Infektionen wirksamen pharmazeutischen Präparaten, dadurch gekennzeichnet, dass ein Cephemderivat der allgemeinen Formel I gegebenenfalls mit pharmazeutisch üblichen Trägerstoffen oder Verdünnungsmitteln in eine pharmazeutisch geeignete Verabreichungsform gebracht wird.

## Claims

1. Cephem derivatives of the general formula I

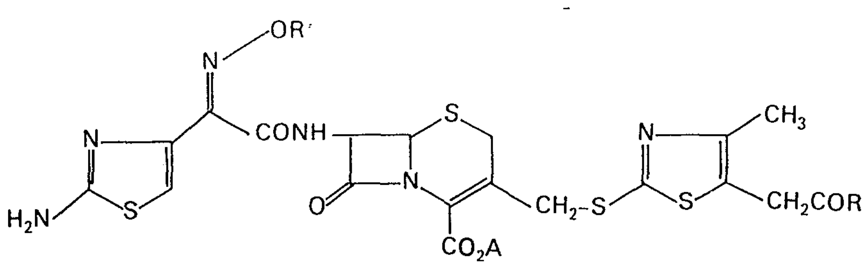

I

in which R denotes
alkoxy with 1-4 C-atoms which may be substituted by alkoxy with 1-4 C-atoms or phenyloxy,
alkenoxy with 2-3 C-atoms which may be substituted by phenyl,
alkinoxy with 2-3 C-atoms,
phenyloxy which may be substituted by halogen, alkyl with 1-4 C-atoms or alkoxy with 1-4 C-atoms, phenylalkoxy with 1-2 C-atoms in the alkyl part, wherein the alkylpart may be substituted by halogen, alkyl with 1-4 C-atoms or alkoxy with 1-4 C-atoms, amino,
alkylamino or dialkylamino with 1-4 C-atoms in each alkyl part or denotes
phenylamino wherein the phenyl part may be substituted by halogen, alkyl with 1-4 C-atoms or alkoxy with 1-4 C-atoms,

R′ represents alkyl with 1-4 C-atoms, and in which A represents hydrogen, a physiologically acceptable cation or a physiologically acceptable ester group,
and in which the R′O group is in the syn-position.

2. A process for the preparation of a cephem derivative of the formula I, which comprises reacting a lactam of the formula II

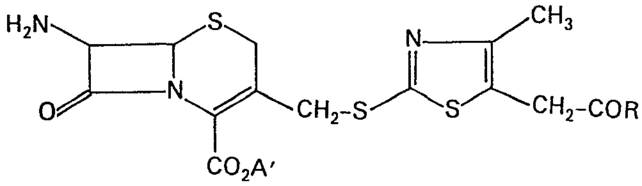

II

in which R has the meanings indicated above and A′ represents a cation, a protective group or a physiologically acceptable ester, with a carboxylic acid of the formula III

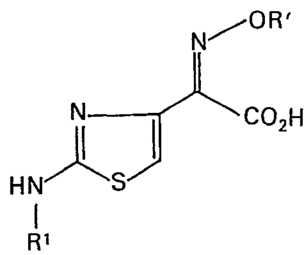

III

or with a reactive derivative thereof, in which $R^1$ represents hydrogen or an amino protective group which is known in peptide chemistry, and $R^1$ has the meanings indicated above to give a compound of the formula IV

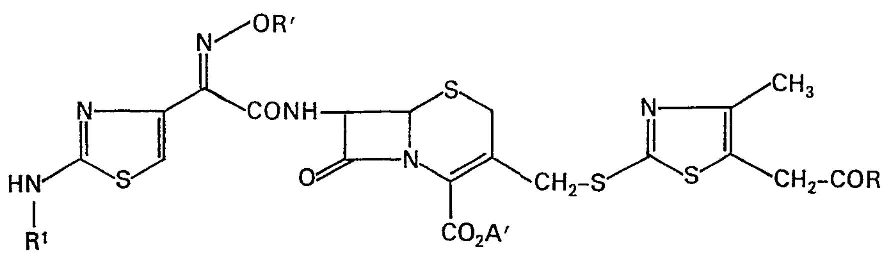

IV

in which $R^1$, R″ and A′ have the abovementioned meanings, or reacting a cephem compound of the formula VI

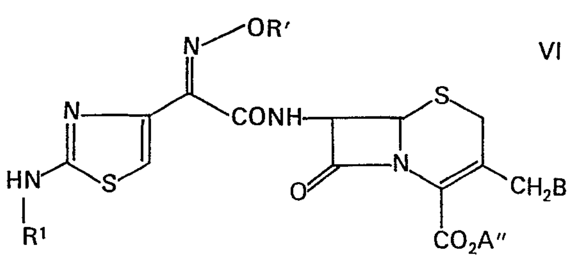

VI

in which $R^1$ and R′ have the abovementioned meaning, A′′ represents hydrogen or a cation and B represents a group which can be replaced by the nucleophilic radical of the compound of the formula V, with a compound of the formula V

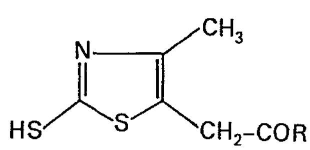

V

in which R has the abovementioned meaning, and, in the resulting compounds, if desired α) splitting off a radical $R^1$ and/or A′ which denote a protective group and β) converting a resulting salt without further treatment or via the free carboxylic acids into a physiologically acceptable ester.

3. Pharmaceutical formulations which are effective against bacterial infections and which contain a cephem derivative of the formula I.

4. A process for the preparation of pharmaceutical formulations which are effective against bacterial infections, wherein a cephem derivative

of the formula I, if desired together with pharmaceutically customary excipients or diluents, is brought into a pharmaceutically suitable administration form.

## Revendications

1. Dérivés de céphème de formule générale I:

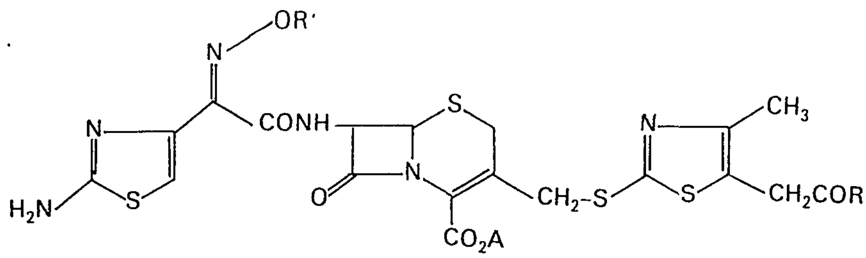

I

dans laquelle R signifie:
un groupe alcoxy à 1-4 atomes de carbone qui peut être substitué par un groupe alcoxy à 1-4 atomes de carbone ou par un groupe phényloxy,
un groupe alcénoxy à 2 ou 3 atomes de carbone, qui peut être substitué par un groupe phényle,
un groupe alcynoxy à 2 ou 3 atomes de carbone,
un groupe phényloxy qui peut être substitué par un halogène, par un groupe alkyle à 1-4 atomes de carbone ou par un groupe alcoxy à 1-4 atomes de carbone,
un groupe phénylalcoxy ayant 1 ou 2 atomes de carbone dans la partie alkyle, la partie phényle pouvant être substituée par un halogène, par un groupe alkyle à 1-4 atomes de carbone ou par un groupe alcoxy à 1-4 atomes de carbone,
un groupe amino,
un groupe alkyle ou dialkylamino ayant de 1 à 4 atomes de carbone dans chaque partie alkyle,
un groupe phénylamino, la partie phényle pouvant être substitué par un halogène, par un groupe alkyle à 1-4 atomes de carbone ou par un groupe alcoxy à 1-4 atomes de carbone, R′ représente un groupe alkyle à 1-4 atomes de carbone et A représente l'hydrogène, un cation physiologiquement acceptable ou un groupe ester physiologiquement acceptable et dans laquelle le groupe R′O est en position syn.

2. Procédé de préparation de dérivés de céphème de formule générale I, caractérisé en ce que l'on fait réagir des lactames de formule générale II:

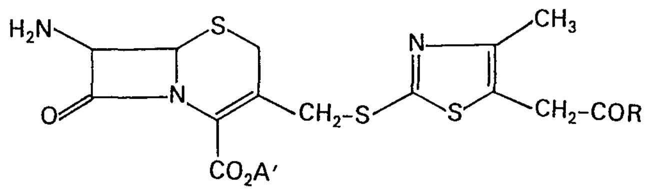

II

dans laquelle R a la signification indiquée ci-dessus et A′ représente un cation, un groupe protecteur ou un ester physiologiquement acceptable, avec un acide carboxylique de formule générale III:

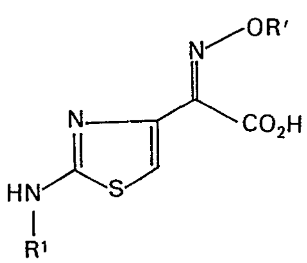

III

ou avec un dérivé réactif de celui-ci, dans lequel $R^1$ représente l'hydrogène ou un groupe protecteur du groupe amino connu dans la chimie des peptides et R′ a la signification ci-dessus, pour donner un composé de formule générale IV:

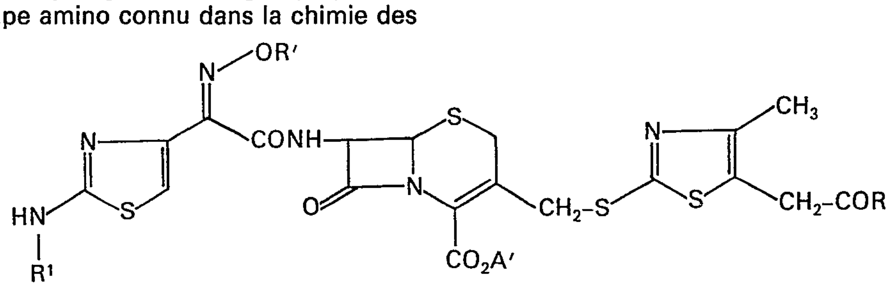

IV

dans laquelle $R^1$, $R'$, R et A ont les significations indiquées ci-dessus, ou bien

on fait réagir des composés de céphème de formule VI:

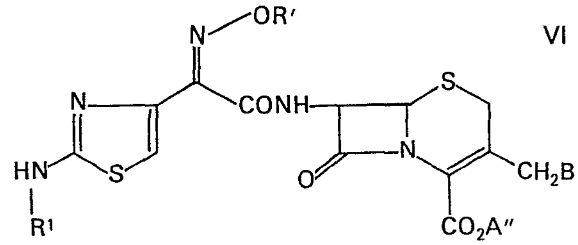

VI

dans laquelle $R^1$ et $R'$ ont la signification indiquée ci-dessus, A″ représente l'hydrogène ou un cation et B représente un groupe qui peut être échangé avec le radical nucléophile du composé de formule V, avec un composé de formule générale V:

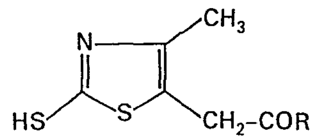

V

dans laquelle R a la signification indiquée ci-dessus et éventuellement, dans les composés obtenus,

α) on enlève un radical $R^1$ et/ou A′ lorsque celui-ci désigne un groupe protecteur et

β) on convertit un sel obtenu, directement ou en passant par les acides carboxyliques libres, en un ester physiologiquement acceptable.

3. Préparation pharmaceutique active contre les infections bactériennes, caractérisée en ce qu'elle contient des dérivés de céphème de formule générale I.

4. Procédé de fabrication de préparations pharmaceutiques actives contre les infections bactériennes, caractérisé en ce que l'on amène un dérivé de céphème de formule générale I, éventuellement avec des supports ou des diluants usuels en pharmacie, à une forme d'administration pharmaceutique appropriée.